**Europäisches Patentamt**

**European Patent Office**

**Office européen des brevets**

(19)

(11) Veröffentlichungsnummer: **0 204 917 B1**

(12) # EUROPÄISCHE PATENTSCHRIFT

(45) Veröffentlichungstag der Patentschrift:
**16.01.91 Patentblatt 91/03**

(21) Anmeldenummer: **86104969.0**

(22) Anmeldetag: **11.04.86**

(51) Int. Cl.$^5$: **C07C 27/12,** C07C 27/26,
C07C 49/403, C07C 35/08,
C07C 45/33, C07C 45/53,
C07C 29/50, C07C 29/132,
C07C 45/80, C07C 45/82

(54) **Verfahren zur Aufarbeitung von Cyclohexanol, Cyclohexanon sowie Cyclohexylhydroperoxid enthaltenden Reaktionsgemischen.**

(30) Priorität: **16.04.85 DE 3513568**

(43) Veröffentlichungstag der Anmeldung:
**17.12.86 Patentblatt 86/51**

(45) Bekanntmachung des Hinweises auf die
Patenterteilung:
**16.01.91 Patentblatt 91/03**

(84) Benannte Vertragsstaaten:
**BE CH DE FR GB IT LI NL**

(56) Entgegenhaltungen:
CH-A- 545 758
FR-A- 1 281 484
FR-A- 1 505 363
FR-A- 2 096 125

(56) Entgegenhaltungen:
FR-A- 2 140 088
FR-A- 2 207 892
FR-A- 2 207 898
GB-A- 1 190 002

(73) Patentinhaber: **BASF Aktiengesellschaft
Carl-Bosch-Strasse 38
D-6700 Ludwigshafen (DE)**

(72) Erfinder: **Hartig, Juergen, Dr.
In der Schleit 9
D-6718 Gruenstadt (DE)**
Erfinder: **Stoessel, Armin, Dr.
Immengaertenweg 20
D-6710 Frankenthal (DE)**
Erfinder: **Lucas, Ekhart, Dr.
Burgunderweg 7
D-6706 Wachenheim (DE)**

## Beschreibung

Aus der EP-A-92 867 ist bekannt, daß man Cyclohexanol, Cyclohexanon sowie Cyclohexylhydroperoxid enthaltende Oxidationsgemische aus der Cyclohexan-Oxidation mit wäßrigen Lösungen von Alkalimetallhydroxiden unter Mitverwendung von katalytisch wirkenden Metallsalzen behandelt, um das im Reaktionsgemisch enthaltene Cyclohexylhydroperoxid in Cyclohexanol und Cyclohexanon umzuwandeln. Dieses Verfahren hat den Nachteil, daß man erhebliche Mengen an Alkalimetallhydroxiden anwenden muß, und zudem beträchtliche Mengen an Cyclohexanon unter Bildung von Hochsiedern verloren gehen.

Nach einem anderen aus der CH-PS 545 758 bekannten Verfahren werden Cyclohexanol Cyclohexanon sowie Cyclohexylhydroperoxid enthaltende Reaktionsgemische aus der Cyclohexan-Oxidation zunächst in Gegenwart eines Edelmetallkatalysators hydriert und anschießend das so erhaltene Hydriergemisch mit wäßrigen Alkalilaugen behandelt. Auch nach diesem Verfahren bilden sich immer noch 2,7 Gew.% hochsiedende Nebenprodukte.

Es war deshalb die technische Aufgabe gestellt, ein Verfahren zur Aufarbeitung von Cyclohexanol, Cyclohexanon sowie Cyclohexylhydroperoxid enthaltenden Reaktionsgemischen aus der Cyclohexanonoxidation zur Verfügung zu stellen, bei dem geringere Mengen Ablaugen als bisher anfallen und insbesondere die Bildung von hochsiedenden Nebenprodukten vermindert wird.

Diese Aufgabe wird gelöst in einem Verfahren zur Aufarbeitung von Cyclohexanol, Cyclohexanon sowie Cyclohexylhydroperoxid enthaltenden Reaktionsgemichen, die bei der Oxidation von Cyclohexan mit molekularem Sauerstoff oder solchen enthaltenden Gasen in flüssiger Phase erhalten worden sind, wobei man das Reaktionsgemisch in Gegenwart von Edelmetallkatalysatoren bei erhöhter Temperatur und unter erhöhtem druck hydriert, mit wäßrigen Alkalilösungen behandelt und Cyclohexanol und Cyclohexanon durch Destillation abtrennt, dadurch gekennzeichnet, daß man

a) das Cyclohexanol, Cyclohexanon und Cyclohexylhydroperoxid enthaltende Reaktionsgemisch mit wäßriger Alkalicarbonatlösung wäscht.

b) das gewaschene Reaktionsgemisch in an sich bekannter Weise in Gegenwart von Edelmetallkatalysatoren bei erhöhter Temperatur und unter erhöhtem Druck hydriert

c) das hydrierte Reaktionsgemisch mit wäßriger Alkalicarbonatlösung behandelt und

d) die anfallenden Alkalicarbonat enthaltenden wäßrigen Ablaugen unter Wiedergewinnung von Alkalicarbonat verbrennt und

e) das gewonnene Alkalicarbonat in Form einer wäßrigen Lösung wiederverwendet.

Das neue Verfahren hat den Vorteil, daß der Anfall an behandlungsbedürftigen Ablaugen vermindert wird. Ferner hat das neue Verfahren den Vorteil, daß der Anfall an hochsiedenden Nebenprodukten vermindert wird.

Erfindungsgemäß geht man von Reaktionsgemischen aus, die durch Oxidation von Cyclohexan mit molekularem Sauerstoff oder solchen enthaltenden Gasen, z.B. Luft in flüssiger Phase erhalten worden sind. Hierbei hält man vorteilhaft Temperaturen von 130 bis 200°C und Drücke von 5 bis 25 bar ein. Gegebenenfalls werden Katalysatoren wie Kobaltsalze mitverwendet. Typische Reaktionsgemische enthalten neben Cyclohexan 3 bis 7 Gew.% Cyclohexanon und Cyclohexanol sowie 0,5 bis 3,5 Gew.% Cyclohexylhydroperoxid, ferner Nebenprodukte wie Ester, Carbonsäuren sowie gegebenenfalls Wasser bis zu 2 Gew.%. Geeignete Reaktionsgemische erhält man beispielsweise nach dem in der DE-PS 10 46 610 beschriebenen Verfahren. Die so erhaltenen Reaktionsgemische werden vor der Weiterbehandlung mit Wasser und/oder wäßrigen Alkalicarbonatlösungen gewaschen.

Das Reaktionsgemisch wird in Gegenwart von Edelmetallkatalysatoren bei erhöhter Temperatur und unter erhöhtem Druck hydriert. Vorteilhaft hält man hierbei eine Temperatur von 80 bis 160°C, insbesondere 100 bis 140°C ein. Vorzugsweise führt man die Hydrierung unter einem Druck von 3 bis 20, insbesondere 5 bis 15 bar durch.

Geeignete Edelmetallkatalysatoren sind z.B. die Metalle Palladium, Platin, Rhodium oder Ruthenium. Besonders bevorzugt ist Palladium. Die katalytisch aktiven Metalle werden vorteilhaft auf Trägern, wie Kohle, Kieselgur, Silikagel oder Aluminiumoxid, niedergeschlagen, verwendet. Solche Trägerkatalysatorer haben vorteilhaft einen Gehalt von 0,1 bis 1,0 Gew.% an den genannten Edelmetallen. Ein besonders bevorzugter Katalysator ist Palladium auf $\gamma$-Aluminiumoxid.

Nach Abtrennen des Wasserstoffs wird das so erhaltene hydrierte Gemisch, das neben Cyclohexan Cyclohexanol und Cyclohexanon sowie Nebenprodukte wie Carbonsäuren und Ester enthält, mit wäßrigen Lösungen von Alkalicarbonaten z.B. Natriumcarbonat oder Kaliumcarbonat behandelt. Kaliumcarbonat hat sich besonders bewährt. Vorteilhaft wendet man je Gewichtsteil hydriertes Reaktionsgemisch 0,05 bis 1,0 Mol Alkalicarbonat in Form einer 5 bis 50 gew.%igen wäßrigen Lösung an. Die Behandlung führt man in der Regel bei einer Temperatur von 80 bis 140°C und unter einem Druck von 5 bis 20 bar durch. Die Behandlung wird unter guter Durchmischung der Phasen, z.B. in einem Mischrohr durchgeführt, und hierbei vorzugsweise eine Verweilzeit von 2 bis 20 Minuten eingehalten. Die wäßrige Alkalicarbonatlösung wird anschließend mit üblichen Trennverfahren, z.B. Dekantieren, von dem so behandelten Hydriergemisch abgetrennt. Vorteilhaft wird die so erhaltene wäßrige Ablauge für die Behandlung des Reaktionsgemisches aus der Cyclohexan-Oxidation vor der Hydrierstufe verwendet.

Das so behandelte Hydriergemisch wird durch Destillation aufgearbeitet, wobei man das gewonnene Cyclohexan wiederum in die Cyclohexan-Oxidation zurückführt und Cyclohexanol und Cyclohexanon gewinnt, die wiederum durch Destillation getrennt werden.

Falls Cyclohexanon das gewünschte Endprodukt ist, wird das erhaltene Cyclohexanol in Gegenwart von Zinkoxidkatalysatoren bei einer Temperatur von 320 bis 390°C in der Gasphase dehydriert. Ein geeignetes Verfahren wird beispielsweise beschrieben in DE-AS 12 11 629.

Vorteilhaft verwendet man den hierbei anfallenden Wasserstoff für die Hydrierung des Reaktionsgemisches aus der Cyclohexan-Oxidation wie ober beschrieben.

Um die Menge an entsorgungsbedürften Ablaugen zu vermindern, werden die wäßrigen Alkalicarbonatlösungen gegebenenfalls zusammen mit anderen Waschwässern und Hochsiedern verbrannt und Alkalicarbonat wiedergewonnen, das wiederum zur Alkalibehandlung in Form von wäßriger Alkalicarbonatlösung wiederverwendet wird.

Die nach dem Verfahren der Erfindung erzeugten Wertstoffe Cyclohexanol und Cyclohexanon sind wichtige Ausgangsstoffe für Faserrohstoffe wie Adipinsäure oder Caprolactam.

Das Verfahren nach der Erfindung sei an folgenden Beispielen veranschaulicht.

Beispiel 1

In einen mit Einbauten versehenen Reaktionsturm werden stündlich 145 kg Cyclohexan und 10,0 Nm³ Luft eingeleitet. Die Umsetzung wird bei einer Temperatur von 170°C und unter einem Druck von 17 bar durchgeführt. Das so erhaltene Reaktionsgemisch wird bei 100°C mit 4 l/h Wasser gewaschen. Die wäßrige Phase wird abgetrennt. Das so erhaltene Reaktionsgemisch enthält neben Cyclohexan 1,45 Gew.% Cyclohexanol, 0,55 Gew.% Cyclohexanon, 3,1 Gew.% Cyclohexylhydroperoxid sowie geringe Mengen an Carbonsäuren und Carbonsäureestern.

Anschließend wird das Reaktionsgemisch zusammen mit einer ca. 50 gew.%igen wäßrigen Kaliumcarbonat-Lösung, die als Ablauge bei der Alkalibehandlung nach der Hydrierung anfällt durch eine Mischstrecke geleitet und nach dem Abtrennen der wäßrigen Phse mit 0,5 l/h Wasser gewaschen, um Kalisalze zu entfernen. Das so vorbehandelte Reaktionsgemisch wird von unten in einen Reaktor von 20 cm Durchmesser und einer Höhe von 1,2 m, der mit 0,25 Gew.% Palladium enthaltenden γ-Aluminiumsträngen beschickt ist, geleitet. Gleichzeitig wird von unten stündlich 5 Nm³ Wasserstoff zugeführt. Die Hydrierung wird bei einer Temperatur von 120°C und unter einem Druck von 8 bar durchgeführt. Hierbei wird Cyclohexylhydroperoxid vollständig in Cyclohexanol und zugleich Cyclohexanon umgewandelt. In einem nachgeschalteten Abscheider trennt man den überstöchiometrisch zugeführten Wasserstoff ab, der wiederum für die Hydrierung verwendet wird. Das bei der Hydrierung anfallende Reaktionswasser wird ebenfalls in diesem Abscheider abgetrennt und nach der Alkalibehandlung als Waschwasser verwendet. Anschließend wird das hydrierte Reaktionsgemisch mit 1,5 kg/h einer 50 gew.%igen wäßrigen Kaliumcarbonat-Lösung in einer Mischstrecke bei einer Temperatur von 120°C und einer Verweilzeit von 10 Minuten behandelt. Anschließend wird die wäßrige Kaliumcarbonat-Lösung abgetrennt und wiederum für die Alkaliwäsche des Rohoxidats vor der Hydrierung verwendet. Zur Entfernung von Kaliumsalzen wird nochmals mit Wasser gewaschen. In einer Kolonne wird durch Destillation Cyclohexan abgetrennt, das wiederum in der Cyclohexan-Oxidation verwendet wird und Cyclohexanol und Cyclohexanon als Wertprodukte gewonnen. Man erhält ein Gemisch aus 76,5 Gew.% Cyclohexanol, 21,0 Gew.% Cyclohexanon, 1,5 Gew.% Leichtsiedern sowie 1,0 Gew.% Hochsiedern. Bezogen auf einen Cyclohexan-Umsatz von 4,60% beträgt die Ausbeute an Cyclohexanon und Cyclohexanol 84,5 Mol.%.

Das nach der Abtrennung des Cyclohexanons durch Destillation verbleibende Cyclohexanol wird in Gegenwart eines Zinkoxid-Katalysators bei einer Temperatur von 365°C in der Gasphase dehydriert. Der hierbei anfallende Wasserstoff wird zur Hydrierung des Reaktionsgemisches aus Cyclohexan-Oxidation verwendet.

Beispiel 2

In dem mit Einbauten versehenen Reaktionsturm werden stündlich 145 kg Cyclohexan und 10,0 Nm³ Luft eingeleitet. Die Umsetzung wird bei einer Temperatur von 176°C und unter einem Druck von 14,8 bar durchgeführt. Das so erhaltene Reaktionsgemisch wird bei 90°C und 4 l/h Wasser gewaschen. Die wäßrige Phase wird abgetrennt. Das so erhaltene Reaktionsgemisch enthält neben Cyclohexan 1,5 Gew.% Cyclohexanol, 0,55 Gew.% Cyclohexanon, 3,1 Gew.% Cyclohexylhydroperoxid sowie geringe Mengen an Carbonsäuren und Carbonsäureestern.

Anschließend wird das Reaktionsgemisch zusammen mit einer ca. 16 gew.%igen wäßrigen Natriumcarbonat-Lösung, die als Ablauge bei der Alkalibehandlung nach der Hydrierung anfällt durch eine Mischstrecke geleitet und nach dem Abtrennen der wäßrigen Phase mit 0,5 l/h Wasser gewaschen, um Natriumsalze zu entfernen. Das so vorbehandelte Reaktionsgemisch wird von unten in einen Reaktor von 20 cm Durchmesser und einer Höhe von 1,2 m, der mit 0,25 Gew.% Palladium enthaltenden γ-Aluminiumsträngenbeschickt ist, geleitet. Gleichzeitig wird von unten stündlich 3 Nn³ Wasserstoff zugeführt. Die Hydrierung wird bei einer Temperatur von 100°C und unter einem Druck von 6,8 bar durchgeführt. Hierbei wird Cyclohexylhydroperoxid vollständig in Cyclohexanol und zugleich -hexanon umgewandelt. In einem nachgeschalteten Abscheider trennt man den über stöchiometrisch zugeführten Wasserstoff ab, der wiederum für die Hydrierung verwendet wird. Das bei der Hydrierung anfallende Reaktionswasser wird ebenfalls in diesem Abscheider abgetrennt und nach der Alkalibehandlung als Waschwasser verwendet. Anschließend wird das hydrierte Reaktionsgemisch mit 8 kg/h einer 16 gew.%igen wäßrigen Natriumcarbonat-Lösung in einer Mischstrecke bei einer Temperatur von 125°C und einer Verweilzeit von 10 Minuten behandelt. Anschließend wird die wäßrige Natriumcarbonat-Lösung abgetrennt und wiederum für die Alkaliwäsche des Rohoxidats vor der Hydrierung verwendet. Zur Entfernung von Natriumsalzen wird nochmals mit Wasser gewaschen. In einer Kolonne wird durch Destillation Cyclohexan abgetrennt, das wiederum in der Cyclohexan-Oxidation verwendet wird und Cyclohexanol und Cyclohexanon als Wertprodukte gewonnen. Man erhält ein Gemisch aus 79,0 Gew.% Cyclohexanol, 18,5 Gew.% Cyclohexanon, 1,2 Gew.% Leichtsiedern sowie 1,3 Gew.% Hochsiedern. Bezogen auf einen Cyclohexan-Umsatz von 4,7% beträgt die Ausbeute an Cyclohexanon und Cyclohexanol 84 Mol.%.

Das nach der Abtrennung des Cyclohexanons durch Destillation verbleibende Cyclohexanol wird in Gegenwart eines Zinkoxid-Katalysators bei einer Temperatur von 365°C in der Gasphase dehydriert. Der hierbei anfallende Wasserstoff wird zur Hydrierung des Reaktionsgemisches aus Cyclohexan-Oxidation verwendet.

## Ansprüche

1. Verfahren zur Aufarbeitung von Cyclohexanol, Cyclohexanon sowie Cyclohexylhydroperoxid enthaltenden Reaktionsgemischen, die bei der Oxidation von Cyclohexan mit molekularem Sauerstoff oder solchen enthaltenden Gasen in flüssiger Phase erhalten worden sind, wobei man das Reaktionsgemisch in Gegenwart von Edelmetallkatalysatoren bei erhöhter Temperatur und unter erhöhtem druck hydriert, mit wäßrigen Alkalilösungen behandelt und Cyclohexanol und Cyclohexanon durch Destillation abtrennt, dadurch gekennzeichnet, daß man

a) das Cyclohexanol, Cyclohexanon und Cyclohexylhydroperoxid enthaltende Reaktionsgemisch mit wäßriger Alkalicarbonatlösung wäscht.

b) das gewaschene Reaktionsgemisch in an sich bekannter Weise in Gegenwart von Edelmetallkatalysatoren bei erhöhter Temperatur und unter erhöhtem Druck hydriert

c) das hydrierte Reaktionsgemisch mit wäßriger Alkalicarbonatlösung behandelt und

d) die anfallenden Alkalicarbonat enthaltenden wäßrigen Ablaugen unter Wiedergewinnung von Alkalicarbonat verbrennt und

e) das gewonnene Alkalicarbonat in Form einer wäßrigen Lösung wiederverwendet.

2. Verfahren nach Anspruch 1, dadurch gekennzeichnet, daß man in Stufe c je Gewichtsteil hydriertes Reaktionsgemisch 0,05 bis 1,0 Mol Alkalicarbonat in Form einer 5 bis 50 gew.-%igen wäßrigen Lösung anwendet.

3. Verfahren nach den Ansprüchen 1 und 2, dadurch gekennzeichnet, daß man in Stufe c die Behandlung mit wäßrigen Alkalicarbonat-Lösungen bei einer Temperatur von 50 bis 140°C durchführt.

4. Verfahren nach den Ansprüchen 1 bis 3, dadurch gekennzeichnet, daß man die in Stufe c anfallende Alkalicarbonat enthaltend Ablauge zum Waschen des Reaktionsgemisches in Stufe a verwendet.

5. Verfahren nach den Ansprüchen 1 bis 4, dadurch gekennzeichnet, daß man Cyclohexanol in Gegenwart von Zinkoxid-Katalysatoren bei einer Temperatur von 320 bis 390°C in der Gasphase zu Cyclohexanon dehydriert und den hierbei anfallenden Wasserstoff für die Hydrierung des Reaktionsgemisches in Stufe b verwendet.

## Claims

1. A process for working up a reaction mixture which contains cyclohexanol, cyclohexanone and cyclohexyl hydroperoxide and is obtained in the oxidation of cyclohexane with molecular oxygen, or with a gas containing same, in the liquid phase, wherein the reaction mixture is hydrogenated in the presence of a noble metal catalyst at elevated temperatures and under superatmospheric pressure, the resulting mixture is treated with aqueous alkali, and cyclohexanol and cyclohexanone are separated off by distillation, which comprises

a) washing the reaction mixture which contains the cyclohexanol, cyclohexanone and cyclohexyl hydroperoxide with an aqueous solution of an alkali metal carbonate,

b) hydrogenating the washed reaction mixture in a conventional manner in the presence of a noble metal catalyst at elevated temperatures and under superatmospheric pressure,

c) treating the hydrogenated reaction mixture with an aqueous solution of an alkali metal carbonate,

d) incinerating the resulting aqueous waste liquor containing an alkali metal carbonate to recover said alkali metal carbonate, and

e) reusing the alkali metal carbonate in the form of an aqueous solution.

2. A process as claimed in claim 1, wherein in step c from 0.05 to 1.0 mole of alkali metal carbonate, in the form of a 5-50% strength by weight aqueous solution, is used per part by weight of hydrogenated reaction mixture.

3. A process as claimed in claim 1 or 2, wherein in step c the treatment with the aqueous alkali metal carbonate solution is carried out at from 50 to 140°C.

4. A process as claimed in any of claims 1 to 3, wherein the alkali metal carbonate waste liquor obtained in stage c is used for washing the reaction mixture in stage a.

5. A process as claimed in any of claims 1 to 4, wherein cyclohexanol is dehydrogenated in the presence of a zinc oxide catalyst at from 320 to 390°C in the gas phase to give cyclohexanone, and the hydrogen obtained in this procedure is used for hydrogenating the reaction mixture in step b.


**Revendications**

1. Procédé de traitement de mélanges réactionnels contenant du cyclohexanol, de la cyclohexanone ainsi que de l'hydroperoxyde de cyclohexyle, qui ont été obtenus par oxydation de cyclohexane avec de l'oxygène moléculaire ou de gaz en contenant, en phase liquide, par hydrogénation du mélange réactionnel en présence de catalyseurs à base de métaux nobles à température élevée et sous pression élevée, traitement par des solutions alcalines aqueuses et séparation du cyclohexanol et de la cyclohexanone par distillation, caractérisé en ce que

a) on lave le mélange réactionnel contenant le cyclohexanol, la cyclohexanone et l'hydroperoxyde de cyclohexyle avec une solution aqueuse de carbonate alcalin,

b) on hydrogène le mélange réactionnel lavé d'une façon connue en soi en présence de catalyseurs à base de métaux nobles à température élevée et sous pression élevée,

c) on traite le mélange réactionnel hydrogéné par une solution aqueuse de carbonate alcalin et

d) on sépare les liqueurs aqueuses contenant du carbonate alcalin accumulé pour récupérer du carbonate alcalin, et

e) on réutilise le carbonate alcalin récupéré sous forme d'une solution aqueuse.

2. Procédé selon la revendication 1, caractérisé en ce qu'on utilise dans l'étape c), par partie en poids de mélange réactionnel, 0,05 à 1,0 mole de carbonate alcalin sous forme d'une solution aqueuse à 5-50% en poids.

3. Procédé selon l'une ou l'autre des revendications 1 et 2, caractérisé en ce que, dans l'étape c), on effectue le traitement par des solutions aqueuses de carbonate alcalin à une température de 50 à 140°C.

4. Procédé selon l'une quelconque des revendications 1 à 3, caractérisé en ce qu'on utilise dans l'étape a), pour laver le mélange réactionnel, les liqueurs contenant du carbonate alcalin obtenues dans l'étape c).

5. Procédé selon l'une quelconque des revendications 1 à 4, caractérisé en ce qu'on déshydrogène en cyclohexanone du cyclohexanol en présence de catalyseurs à l'oxyde de zinc à une température de 320°C à 390°C en phase gazeuse et on utilise l'hydrogène ainsi formé pour l'hydrogénation du mélange réactionnel dans l'étape b).